# EUROPÄISCHE PATENTSCHRIFT

(11) **EP 2 016 342 B1**
(45) Veröffentlichungstag und Bekanntmachung des Hinweises auf die Patenterteilung: **23.12.2015**
(21) Anmeldenummer: 07724708.8
(22) Anmeldetag: 28.04.2007
(51) Int. Cl.: F24F 3/16, A61L 9/20, G10K 11/16, F24F 13/24

(54) **BAUTEIL ZUR SCHALLABSORPTION UND LUFTAUFBEREITUNG**
COMPONENT FOR SOUND ABSORPTION AND AIR CONDITIONING
COMPOSANT POUR L'ABSORPTION DE BRUIT ET LE TRAITEMENT DE L'AIR

(30) Priorität: 11.05.2006 DE 102006022083
(43) Veröffentlichungstag der Anmeldung: 21.01.2009
(73) Patentinhaber: Fraunhofer-Gesellschaft zur Förderung der angewandten Forschung e.V., 80686 München (DE)
(72) Erfinder: LEISTNER, Philip, 70569 Stuttgart (DE); BREUER, Klaus, 83229 Aschau (DE); SEDLBAUER, Klaus, 83607 Holzkirchen (DE)
(86) Internationale Anmeldenummer: PCT/EP2007/003781
(87) Internationale Veröffentlichungsnummer: WO 2007/131614

(56) Entgegenhaltungen:
- EP-A1- 1 527 809
- JP-A- 10 277 366
- JP-A- 2000 257 185
- JP-A- 2000 279 762
- JP-A- 2001 271 313
- JP-A- 2002 244 664
- US-A1- 2004 166 037

## Beschreibung

### Technisches Gebiet

Die Erfindung betrifft ein Bauteil zur Schallabsorption und Luftaufbereitung, mit dem die akustischen und thermischen Raumeigenschaften sowie die Beleuchtungs- und Luftqualität in Räumen und Verkehrsmitteln gesteuert werden.

### Stand der Technik

In der Akustik, insbesondere in der Raumakustik, ist eine Vielzahl von Konzepten und Bauteilen zur Schallabsorption bekannt. In den letzten Jahren wurden erhebliche Fortschritte erzielt, Bauteile zu schaffen, welche einen geringen Raum- und Materialbedarf haben und in die gewünschte architektonische Raumgestaltung integrierbar sind.

Eine Gruppe dieser Bauteile sind mikroperforierte Absorber. Aus der DE 197 54 107 C1 ist z.B. ein mikroperforierter Schallabsorber, bestehend aus mikroperforierten Folien oder dünnen Platten bekannt. Dabei sind mehrere Folien oder Platten in beliebiger Anordnung zueinander vorgesehen, welche im Raum waagrecht oder schräg aufgehängt sind. Eine Erweiterung der Funktionalität ergibt sich, wenn diese Platten Teile von Lüftungskanälen sind, wie z.B. in DE 197 30 355 C1 beschrieben. In transparenter Ausführung nach DE 43 15 759 C1 ergeben sich ebenfalls zusätzliche Gestaltungsmöglichkeiten, wie z.B. als Vorsatzelemente vor Fenstern aber auch vor Leuchten. Bislang unbekannt sind Verknüpfungen, die zur gleichzeitigen Steuerung anderer Raumeigenschaften, wie Raumlufttemperatur und Raumluftqualität dienen. Um Räume insgesamt behaglich zu gestalten, ist es aber neben der akustischen Konditionierung auch notwendig, diese Parameter zu regulieren. Gerade die Entfernung unerwünschter Luftfremdstoffe (Schadstoffe) oder unerwünschter (flüchtiger organischer Geruchs-) Stoffe aus der Luft ist eine dringende Aufgabe, die allein durch Luftzirkulation nur schwer zu erreichen ist. Natürlich sind zahlreiche Arten der Raumlüftung bekannt, an die allerdings auch wirtschaftliche Ansprüche gestellt werden, und sie sollten möglichst mit einem Minimum an Platz auskommen. Eine spezielle Form zur kombinierten Beeinflussung von Raumakustik und Raumluftqualität wird in DE 101 49 414 A1 beschrieben. Dabei werden gelochte Gipsfaser- oder Gipskartonplatten mit Behältnissen oder flächigen Elementen belegt, die auf Grund ihrer faserigen oder porösen Art Schall absorbieren und sich bei entsprechender Materialwahl (z.B. Zeolith) auch zur passiven Luftreinigung (Einlagerung von Schadstoffen) eignen. Abgesehen von der Frage der Erschöpfung der luftreinigenden Wirkung dieser passiven Effekte unterscheiden sie sich von den so genannten semi-aktiven Prinzipien (Photokatalyse) einerseits stofflich (z.B. Titandioxid als Additiv) und andererseits durch die Unabhängigkeit von UV-Licht. UV-Licht (Tageslicht) ist eine Voraussetzung für die Photokatalyse, die z.B. bei angereicherten Wandbeschichtungen (Textilien, Tapeten) angewendet bzw. ausgenutzt wird. Die Defizite all dieser Bauteile sind die eingeschränkte Gestaltungsvielfalt, die fehlende Multifunktionalität und damit die Anwendbarkeit in Innenräumen, auch angesichts des minimalen zur Verfügung stehenden Platzangebots.

In der DE 693 24 574 T2 wird ein Luftbehandlungsverfahren für Räume auf der Basis von Photokatalyse beschrieben. Dazu wird in einem Raum eine elektrische Allzwecklampe angebracht. Ferner wird eine Dünnschicht eines Photokatalysators aus Feststoffhalbleitermaterial zumindest auf einem Teil der innenfläche des Raums angebracht. Dünnschicht und Lampe sind so angeordnet, dass die Dünnschicht von der Lampe beleuchtet wird. Im Wellenlängenbereich zwischen 300 nm und der Bandabstandsenergie des Feststoffhalbleitermaterials beträgt die auf die Dünnschicht auftreffende Lichtstärke 0,001 - 1 mW/cm². Es ist ersichtlich, dass bei diesem Ansatz der Raumluftaufbereitung hinzunehmen ist, dass die die Dünnschicht sichtbar ist und der Raum beleuchtet werden muss.

Aus der US 2004/01666037 A1 ist ein System zum Filtern von Druckluft bekannt. Das System enthält einen ersten Partikelfilter. Stromabwärts von diesem Filter befinden sich eine Ultraviolettlampe und ein permeabler Reaktionsfilter. Auf diesem Reaktionsfilter ist ein Trägerelement enthalten. Auf diesem Trägerelement sind Titandioxid-Partikel gebunden. Damit ist photokatalytische Behandlung der Luft möglich. Weiterhin sind Filter aus Fasermaterial angeordnet. Diese haben neben der im Vordergrund stehenden Filterwirkung auch eine schallabsorbierende Wirkung.

Aus der JP 2000 257 185 ist eine panelförmige Anordnung bekannt. Diese weist eine Metallplatte mit einer Vielzahl von Öffnungen nach Art eines Lochblechs auf. Diese Metallplatte ist in einer Metallstruktur gelagert. Innerhalb der zur Rückseite geschlossenen und auf der Vorderseite durch die Metallplatte abgeschlossenen Metallstruktur befindet sich ein tuchartiges schallabsorbierendes Material. Bei dem tuchartigen schallabsorbierenden Material handelt es sich um ein Textilerzeugnis, das gewebt sein kann. Es dient als Träger für eine photokalalytische Substanz mit einer Flächendichte von 1 bis 200 g/m². Es sind Lichtquellen zur Bestrahlung des tuchartigen Materials vorhanden. Das tuchartige Material hat eine einstellbare Luftdurchlässigkeit.

### Beschreibung

Aufgabe der vorliegenden Erfindung ist es eine verbesserte, Platz sparende und an eine gewünschte Raumgestaltung anpassbare Möglichkeit zu schaffen, den Schall zu dämpfen, die Temperatur zu beeinflussen und die Luftqualität zu verbessern. Diese Aufgabe wird durch das Bauteil nach den Merkmalen des Anspruches 1 gelöst. Vorteilhafte Ausgestaltungen finden sich in den Unteransprüchen. Zur Lösung des Problems wird ein Bauteil zur Schallabsorption und zur Luftaufbereitung, insbesondere zur Raumluftaufbereitung, vorgeschlagen. Das Bauteil weist ein schallabsorbierendes, mit photokatalytisch aktivem Material beschichtetes Flächengebilde auf, das von UV-Lichtquellen bestrahlbar ist. Es zeichnet sich dadurch aus, dass entlang dem Flächengebilde und/oder durch das Flächengebilde die Luft geführt werden kann. Dabei ist unter einem Flächengebilde nicht nur ein Gebilde mit einer ebenen Fläche zu verstehen. Es kann sich selbstverständlich auch um beliebige andere Flächen, wie etwa gekrümmte oder gewölbte Flächen handeln. Im Allgemeinen wird die Fläche des Flächengebildes der Form der Raumumschließungsfläche angepasst sein.

Das Bauteil zeichnet sich weiterhin durch eine dem Raum zugewandte Vorderseite und eine dem Raum abgewandte Rückseite aus, wobei die Rückseite mit photokatalytisch aktivem Material beschichtet ist. Mit Raum ist hier derjenige Raum gemeint, in dem die Luft aufzubereiten und in dem der Schallpegel zu reduzieren Ist. Es handelt sich nicht um den Raum, der sich zwischen der Rückseite des Bauteils und einer Raumumschließungsfläche, etwa einer Wand, befindet. Damit können die UV-Lichtquellen und das photokatalytisch aktive Material auf der Rückseite für eine im Raum befindliche Person unsichtbar angeordnet werden, wodurch architektonische Anforderungen erfüllt werden können. Ferner ist es möglich die aufzubereitende Luft an der Rückseite vorbeiströmen zu lassen, wodurch ein höherer Anteil der Luft an der Fläche vorbeiströmt als an der Vorderseite, da dort ein größerer Raum vorhanden ist. Zudem kann mit einem derartigen Bauteil mit niedrigem Platzbedarf sowohl die Funktion der Schallabsorption als auch der Luftaufbereitung erzielt werden.

Ein geeignetes Schall absorbierendes Flächengebilde ist eine mikroperforierte Platte, Folie oder Membran oder ein mikroperforierter Plattenabsorber. Derartige mikroperforierte Bauteile haben sich zur Schallabsorption bewährt. Bei dem Bauteil, welches sowohl zur Schallabsorption als auch zur Raumluftaufbereitung dienen soll, hat ein mikroperforiertes schallabsorbierendes Flächengebilde den vorteil, dass die aufbereitete Raumluft durch die Mikroperforation hindurch in den Raum strömen kann.

Ein geeignetes photokatalytisch aktives Material besteht beispielsweise aus der photokatalytisch aktiven Form des Titandioxids. Es ist auch möglich, dass es noch weitere Bestandteile aufweist und dieses Titandioxid lediglich enthält. Die photokatalytisch aktive Form des Titandioxids hat sich als zur Luftaufbereitung geeignet bewährt.

Um die Wirksamkeit des Bauteils zur Raumluftaufbereitung zu erhöhen, kann eine Erhöhung der photokatalytisch wirksamen Oberfläche erreicht werden, indem diese strukturiert ausgebildet ist und dafür beispielsweise Stege oder Sicken aufweist. Damit kann bei allenfalls geringer Erhöhung des Platzbedarfs des Bauteils seine Funktionalität erhöht werden, bzw. bei gleicher Funktionalität ein kleineres Bauteil verwendet werden. Entsprechend kann die gleiche Funktionalität auch kostengünstiger erreicht werden.

Zur Verbesserung der Raumluftaufbereitung kann eine aktive Zuführung der aufzubereitenden Luft in den Rückraum hinter dem schallabsorbierenden Flächengebilde mittels Vorrichtungen zur Luftförderung, beispielsweise mit Ventilatoren, erreicht werden. Der mit Vorrichtungen zur Luftförderung häufig verbundene Nachteil der erhöhten Geräuschentwicklung ist beim erfindungsgemäßen Bauteil weniger gravierend, da ja eine Schallabsorption vorgesehen ist.

Zusätzliche Öffnungen im schallabsorbierenden Flächengebilde senken die Schallabsorption nur in relativ geringen Umfang. Der bei der Durchströmung des Flächengebildes mit der aufbereiteten Raumluft auftretende Durchströmungswiderstand wird durch die Öffnungen aber deutlich reduziert.

Als UV-Lichtquellen eignen sich beispielsweise UV-Leuchtdioden. Diese liefern bei niedrigem Energiebedarf ausreichend UV-Strahlung zur photokatalytischen Raumluftaufbereitung. Verwendet man UV-Lichtquellen, die zugleich sichtbares Licht emittieren, kann das sichtbare Licht zur direkten oder indirekten Beleuchtung benutzt werden. Damit können die Anforderungen der Raumluftaufbereitung und der Beleuchtung mit preisgünstigen Lichtquellen bei relativ niedrigem Energieaufwand erfüllt werden. Bei dieser Ausgestaltung sollte ein transparentes Flächengebilde zum Einsatz kommen. Damit kann zumindest durch diese Bereiche, an denen kein photokatalytisch aktives Material aufgebracht ist, das Licht in den Raum dringen.

Wenn das schallabsorbierende Flächengebilde beheizbar und/oder kühlbar ist oder über eine innere beheizbare oder kühlbare Schicht verfügt, so kann das Bauteil auch zur Klimatisierung des Raums verwendet werden. Es kann also eine weitere Funktion ohne nennenswert erhöhten Platzbedarf des Bauteils erreicht werden.

Durch Verwendung eines transparenten schallabsorbierenden Flächengebildes, auf dessen Rückseite das photokatalytisch aktive Material teilflächig aufgetragen ist, können entsprechende architektonische Bedürfnisse erfüllt werden. Dabei bleibt die gewünschte Funktionalität erhalten.

Zur Verbesserung der Raumluftaufbereitung kann die der Rückseite des schallabsorbierenden Flächengebildes gegenüber liegende Raumbegrenzungsfläche ebenfalls mit photokatalytischem Material beschichtet sein. Damit steht eine wesentlich erhöhte wirksame Fläche zur photokatalytischen Aufbereitung zur Verfügung. Wird die Raumbegrenzungsfläche als reflektierende Fläche ausgebildet, so trifft auf die photokatalytisch aktive Rückseite des schallabsorbierenden Flächengebildes eine erhöhte UV-Strahlung auf, ohne dass leistungsfähigere und damit in der Anschaffung und/oder Betrieb teurere UV-Lichtquellen verwendet werden müssen.

Das Bauteil eignet sich auch gut als Teil eines Kanals oder Raumes einer Lüftungsanlage. Damit trägt die aufbereitete Luft, welche dem Raum zugeführt wird, nicht soviel Schall mit in den Raum hinein. Damit wird in dem Kanal oder dem Raum der Lüftungsanlage ein Schalldämpfer verwirklicht, mit dem zugleich eine Luftaufbereitung möglich ist.

Da sich das Bauteil zur Raumluftaufbereitung und Schallabsorption in Räumen eines Gebäudes eignet, ist es günstig, wenn es an Raumbegrenzungsflächen in Gebäuden anbringbar oder angebracht ist.

Luftaufbereitung und Schallabsorption spielen insbesondere auch in Innenräumen von Verkehrsmitteln eine Rolle. Hier ist es günstig, wenn das Bauteil beispielsweise als Teil der Verkleidung anbringbar oder angebracht ist. Entsprechendes gilt für den Motorraum von Verkehrsmitteln, insbesondere wenn diese von Personen begangen werden, für die im Motorraum eine aufbereitete Luft vorhanden sein muss. Auch wenn ein vollständiger Abschluss des Motorraums zur Umgebung nicht möglich ist, so dass ein Luftaustausch unvermeidlich ist, ist es günstig, wenn die Luft im Motorraum aufbereitet wird. Somit wird vermieden, dass beim unvermeidlichen Luftaustausch schlechte Luft an die Umgebung abgegeben wird. Dies gilt vor allem dann, wenn es nicht möglich ist Luft kontrolliert auszutauschen und nur die ausgetauschte Luft aufzubereiten. Bei der Geräuschentwicklung von Verkehrsmitteln ist Schallsschutz in der Regel erforderlich.

Das Bauteil eignet sich auch als Teil einer Kapselung oder Einhausung für Maschinen oder Geräte. Dies ist vor allem der Fall, wenn Schalldämpfung erforderlich ist und zugleich unkontrollierter Luftaustausch mit der Umgebung unvermeidlich ist. Die oben genannten Überlegungen für Motorräume von Verkehrsmitteln gelten entsprechend.

Nachfolgend wird anhand der Figuren die Erfindung näher beschrieben. Dabei zeigen:
- Fig. 1: Schematische Darstellung des schallabsorbierenden Flächengebildes (1), z.B. als mikroperforierte Platte, dessen Rückseite (2) mit einer Beschichtung aus photokatalytisch aktivem Material versehen ist und durch UV-Lichtquellen (3) bestrahlt wird und an der die Luft (6) des Raumes (5) vorbeigeführt wird.
- Fig. 2: Schnittdarstellung eines Ausschnitts des schallabsorbierenden Flächengebildes (1), dessen Rückseite (2) und dessen Öffnungen mit einer Beschichtung aus photokatalytisch aktivem Material versehen sind
- Fig. 3: Schnittdarstellung eines Ausschnitts des schallabsorbierenden Flächengebildes (1) in strukturierter Form, z.B. mit rückseitigen Stegen, mit photokatalytisch aktiver Beschichtung der Rückseite (2)
- Fig. 4: Schnittdarstellung eines Ausschnitts des schallabsorbierenden Flächengebildes (1) mit einer inneren beheizbaren oder kühlbaren Schicht (7).
- Fig. 5: Schematische Darstellung des schallabsorbierenden Flächengebildes (1) als Teil eines Kanals (8).

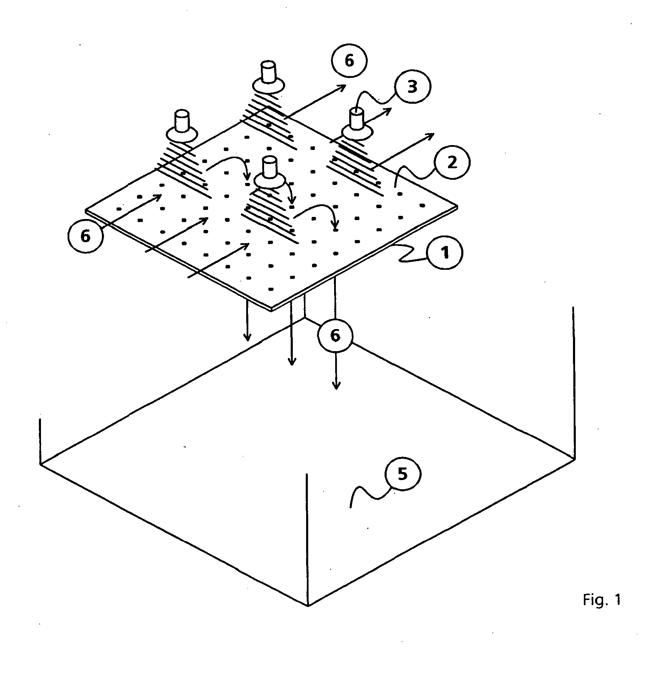

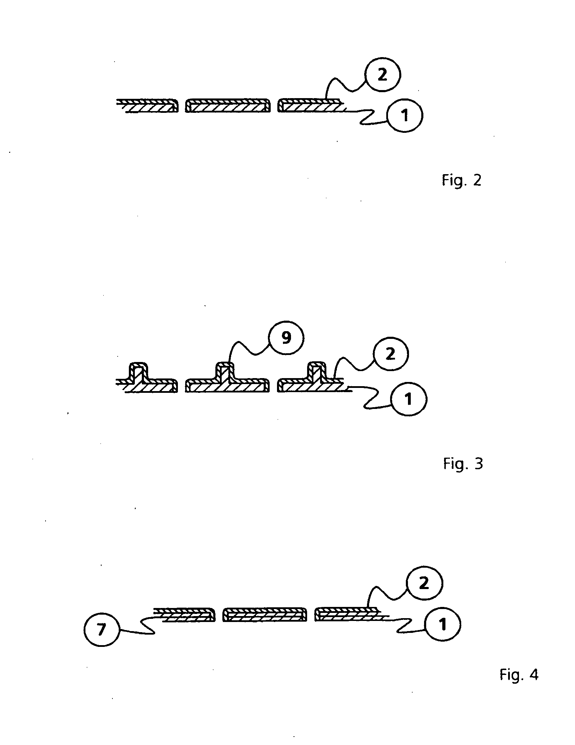

Erfindungsgemäß wurde erkannt, dass es möglich ist, den für die akustische Wirkung erforderlichen Rückraum eines dünnen schallabsorbierenden Flächengebildes (1), z.B. bestehend aus mikroperforierten Platten, für die Funktion der aktiven Verbesserung der Luftqualität (Reinigung) zu nutzen, Fig. 1. Dazu sind die Rückseite (2) und die Öffnungen (Wandungen der Mikrolöcher) des Flächengebildes (1) mit einer Beschichtung aus photokatalytisch aktivem Material, z.B. aus der photokatalytisch aktiven Form des Titandioxids, zu versehen, Fig. 2. Weist die Beschichtung eine gewissen Porosität oder Rauhigkeit auf, trägt dies zur Steigerung der schallabsorbierenden Wirkung der Mikroperforation bei. Zugleich trägt eine solche Beschichtung zur Selbstreinigung der Öffnungen bei.

Zur Aktivierung der Luftreinigung wird Luft (6) aus dem Raum (5) durch den Rückraum des Flächengebildes (1) und gegebenenfalls durch dessen Öffnungen geführt und zugleich dessen beschichtete Rückseite (2) von künstlichen UV-Lichtquellen (3), z.B. vorzugsweise UV-Leuchtdioden, bestrahlt. Die Verwendung von wenige Millimeter großen UV-Leuchtdioden verspricht eine besonders platz sparende Ausführung der Bauteile. Zur Erhöhung der photokatalytisch wirksamen Oberfläche kann das Flächengebilde (1) entsprechend strukturiert sein, z.B. in Gestalt von Stegen oder Sicken, Fig. 3. Dem gleichen Ziel dient eine Beschichtung der anderen, den Rückraum hinter dem Flächengebilde (1) begrenzenden Oberfläche mit photokatalytisch aktivem Material oder deren Ausbildung als (Licht) reflektierende Fläche, z.B. in Form einer verspiegelten Fläche.

Zur Erfüllung einer weiteren Raumfunktion können die Lichtquellen (3) zur direkten oder indirekten Beleuchtung benutzt werden. Ist eine Nutzung der Lichtquellen (3) auch zu direkten Beleuchtungszwecken erwünscht, kann ein transparentes mikroperforiertes Material für das Flächengebilde (1) gewählt werden und die Beschichtung der Rückseite (2) erfolgt nur teilflächig. Die Durchlässigkeit für den UV-Anteil des Lichts kann ebenfalls durch Materialwahl sichergestellt werden. Der durch die Mikrolöcher dringende Anteil ist angesichts des Lochflächenanteils von bis zu unter einem Prozent vernachlässigbar.

Die Luftführung kann bei geeigneter Positionierung im Raum (5) durch die gegebene Raumluftströmung angetrieben oder durch Vorrichtungen zur Luftförderung (4), z.B. Ventilatoren, unterstützt werden. Ist eine Durchströmung der Öffnungen des Flächengebildes (1) vorgesehen, können sich zusätzliche Lüftungsöffnungen im Flächengebilde (1) als sinnvoll erweisen, um diesen Lufttransport mit niedrigerem Druckverlust zu bewerkstelligen.

Die besondere Freizügigkeit bei der Materialwahl (Metall, Kunststoff) für mikroperforierte Flächengebilde (1) bietet beste Voraussetzungen für eine geometrische Formbarkeit und für eine optimale Beschichtung der Rückseite (2). Zugleich erlaubt sie die einfachstmögliche Integration einer weiteren Funktion: der thermischen Konditionierung (Heizung, Kühlung). Dazu kann ein z.B. metallisches mikroperforiertes Flächengebilde (1) als beheizte oder gekühlte Platte verwendet werden. Als Alternative dazu kann eine deutlich weniger als einen Millimeter dünne Innenschicht (7), Fig. 4, zwischen zwei deckungsgleichen Teilen des mikroperforierten Flächengebildes (1) fungieren. In diesem Fall ergibt sich - abzüglich eines Farbauftrags - eine vierschichtige Anordnung, deren Dicke allerdings problemlos unter einem Millimeter liegen kann.

Die thermische Funktion des Flächengebildes (1) wirkt sich in zweierlei Hinsicht positiv aus. Erstens trägt sie zur thermischen Konditionierung des Raumes (5) bei, und zweitens erhöht sie die Wirksamkeit der aktiven Luftreinigung.

Die thermisch wirksame Innenschicht (7) kann auch aus einer Membran oder Folie mit integrierten Heizwendeln bestehen, wobei hierbei auf planparallele Oberflächen zu achten ist. Im Sinne einer Kosten sparenden und präzisen Fertigung ist es vorteilhaft, die Mikroperforation des Flächengebildes (1) nach Zusammenführung der einzelnen Schichten vorzunehmen.

Insgesamt handelt sich also nicht etwa nur um die Addition von Funktionen, sondern vielmehr um die gezielte Nutzung und Integration von Wechselwirkungen zwischen den Gestaltungsmerkmalen der einzelnen Funktionen. Diese sind mit getrennten Funktionsbauteilen nicht zu erreichen. Es ist von Bedeutung, durch die Kombination eine Reduktion der Montagekosten zu erzielen. Ferner treten bestimmte Anforderungen z.B. an die Schallabsorption und die Luftaufbereitung häufig zusammen auf. So können Maschinen, die eine spezielle Schalldämpfung erfordern, auch eine spezielle Raumluftaufbereitung erfordern. Es können damit von einer Maschine ausgehende Probleme des Schalls und der Luftverschlechterung mit einem Bauteil gelöst werden.

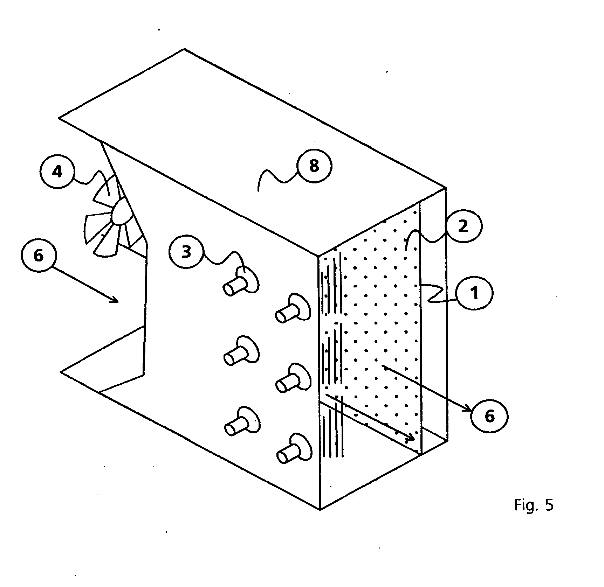

Geeignete Ausführungsformen für das dünne schallabsorbierende Flächengebilde (1) sind auch Folienabsorber oder sogar unperforierte Plattenabsorber. Durch die aktive Zuführung der aufzubereitenden Luft kann die Menge der aufzubereitenden Luft deutlich erhöht werden. Die grundsätzlich nachteiligen Strömungsgeräusche z.B. des Ventilators werden im Rückraum des Flächengebildes (1) durch dessen schallabsorbierende Wirkung reduziert, so dass dieser übliche Nachteil aktiv durchströmter Einrichtungen zur Luftaufbereitung bei der Kombination mit einem Schallabsorber entfällt. Dieser Zusammenhang kann auch regelrecht ausgenutzt werden, wenn Bauteil und Flächengebilde (1) Teile eines Kanals (8), z.B. Schalldämpfer, oder Teilraums einer Lüftungsanlage sind, Fig. 5.

Die weitgehend freie Formbarkeit des Flächengebildes (1) eröffnet Anwendungen sowohl in Gebäuden als auch in Verkehrsmitteln.

## Patentansprüche

1. Bauteil zur Schallabsorption und Luftaufbereitung mit einem schallabsorbierenden, mit phatokatalytisch aktivem Material beschichteten Flächengebilde (1), das von UV-Lichtquellen (3) bestrahlbar ist, wobei entlang dem Flächengebilde (1) und/oder durch das Flächengebilde (1) die Luft geführt werden kann, wobei das Bauteil eine einem Raum (5) zugewandte Vorderseite und eine dem Raum abgewandte Rückseite aufweist, wobei die Rückseite mit photokatalytisch aktivem Material beschichtet ist. **dadurch gekennzeichnet, dass** das schallabsorbierende Flächengebilde (1) eine mikroperforierte Platte, mikroperforierte Folie oder mikroperforierte Membran oder ein mikroperforierter Plattenabsorber ist.

2. Bauteil nach Anspruch 1, **dadurch gekennzeichnet, dass** das photokatalytisch aktive Material aus der photokatalytisch aktiven Form des Titandioxids besteht oder diese enthält.

3. Bauteil nach einem der vorhergehenden Ansprüche, **dadurch gekennzeichnet, dass** das schallabsorbierendes Flächengebilde (1) oder dessen Rückseite (2) zur Erhöhung der photokatalytisch wirksamen Oberfläche strukturiert ausgebildet ist und dafür Stege oder Sicken aufweist.

4. Bauteil nach einem der vorhergehenden Ansprüche, **dadurch gekennzeichnet, dass** mittels Vorrichtungen zur Luftförderung (4), z.B. Ventilatoren, eine aktive Zuführung der aufzubereitenden Luft in den Rückraum hinter dem schallabsorbierenden Flächengebilde (1) erfolgen kann.

5. Bauteil nach einem der vorhergehenden Ansprüche, **dadurch gekennzeichnet, dass** das schallabsorbierende Flächengebilde (1) zusätzliche Öffnungen aufweist, um bei Durchströmung einen geringeren Druckverlust zu gewährleisten.

6. Bauteil nach einem der vorhergehenden Ansprüche, **dadurch gekennzeichnet, dass** die UV-Lichtquellen (3) z.B. UV-Leuchtdioden sind oder die UV-Lichtquellen (3) zugleich sichtbares Licht zur direkten oder indirekten Beleuchtung abstrahlen.

7. Bauteil nach einem der vorhergehenden Ansprüche, **dadurch gekennzeichnet, dass** das schallabsorbierende Flächengebilde (1) beheizbar und/oder kühlbar ist oder über eine innere, beheizbare oder kühlbare Schicht (7) verfügt.

8. Bauteil nach einem der vorhergehenden Ansprüche, **dadurch gekennzeichnet, dass** das schallabsorbierende Flächengebilde (1) transparent ist und auf dessen Rückseite (2) das photokatalytisch aktive Material teilflächig aufgetragen ist.

9. Bauteil nach einem der vorhergehenden Ansprüche, **dadurch gekennzeichnet, dass** die der Rückseite (2) des schallabsorbierenden Flächengebildes (1) gegenüberliegende Raumbegrenzungsfläche ebenfalls mit photokatalytisch aktivem Material beschichtet oder als reflektierende Fläche z.B. verspiegelt ist.

10. Bauteil nach einem der vorhergehenden Ansprüche, **dadurch gekennzeichnet, dass** das Bauteil Teil eines Kanals (8) oder Raumes einer Lüftungsanlage ist.

11. Bauteil nach einem der vorhergehenden Ansprüche, **dadurch gekennzeichnet, dass** das Bauteil an Raumbegrenzungsflächen in Gebäuden anbringbar oder angebracht ist.

12. Bauteil nach einem der Ansprüche 1 bis 10, **dadurch gekennzeichnet, dass** das Bauteil im Innenraum oder Motorraum von Verkehrsmitteln, z.B. als Teil der Verkleidung anbringbar oder angebracht ist.

13. Bauteil nach einem der Ansprüche 1 bis 10, **dadurch gekennzeichnet, dass** das Bauteil Teil einer Kapselung oder Einhausung für Maschinen oder Geräte ist.

## Claims

1. Component for sound absorption and air conditioning with a sound-absorbing planar structure (1) coated with photocatalytically active material, which planar structure can be irradiated by UV light sources (3), wherein the air can be guided along the planar structure (1) and/or through the planar structure (1), wherein the component has a front side facing towards a room (5) and a rear side facing away from the room, the rear side being coated with photocatalytically active material, **characterized in that** the sound-absorbing plantar structure (1) is a microperforated plate, film or membrane or a plate absorber..

2. Component according to claim 1, **characterized in that** the photocatalytically active material comprises or contains the photocatalytically active form of titanium dioxide.

3. Component according to one of the preceding claims, **characterized in that** the sound-absorbing planar structure (1) or the rear side (2) thereof is embodied in a structured manner to increase the photocatalytically active surface and has webs or ribs for this purpose.

4. Component according to one of the preceding claims, **characterized in that** an active supply of the air to be conditioned to the rear space behind the sound-absorbing planar structure (1) can be achieved by means of devices for air conveying (4), for sample, ventilators.

5. Component according to one of the preceding claims, **characterized in that** the sound-absorbing planar structure (1) has additional openings in order to ensure a lower pressure loss with flow-through.

6. Component according to one of the preceding claims, **characterized in that** the UV light sources (3) are, e.g., UV light-emitting diodes or the UV light sources (3) at the same time emit visible light for direct or indirect lighting.

7. Component according to one of the preceding claims, **characterized in that** the sound-absorbing planar structure (1) can be heated and/or cooled or has an inner layer (7) that can be heated or cooled.

8. Component according to one of the preceding claims, **characterized in that** the sound-absorbing planar structure (1) is transparent and on the rear side (2) of which the photocatalytically active material is applied on some parts.

9. Component according to one of the preceding claims, **characterized in that** the room-delimiting surface lyiung opposite the rear side (2) of the sound-absorbing planar structure (1) is likewise coated with photocatalytically active material or is, e.g., mirrored as a reflective surface.

10. Component according to one of the preceding claims, **characterized in that** the component is part of a channel (8) or chamber of a ventilation system.

11. Component according to one of the preceding claims, **characterized in that** the component can be attached or is attached to room-delimiting surfaces in buildings.

12. Component according to one of claims 1 through 10, **characterized in that** the component can be attached or is attached in the interior space or engine comportment of means of transport, e.g., as part of the cladding.

13. Component according to one of claims 1 through 10, **characterized in that** the component is part of an enclosure or housing for machines or equipment.

## Revendications

1. Composant pour l'absorption des sons et le traitement de l'air comprenant une structure de surface (1) absorbant les sons, revêtue d'un matériau photocatalytiquement actif, qui peut être irradié par des sources de lumière UV (3), l'air pouvant être guidé le long de la structure de surface (1) et/ou à travers la structure de surface (1), le composant présentant un côté avant tourné vers un espace (5) et un côté carrière opposé à l'espace, le côté arrière étant revêtu d'un matériau photocatalytiquement actif, **caractérisé en ce que** la structure de surface (1) absorbant les sons est une plaque microperforée, un film microperforé ou une membrane microperforée ou un absorbeur à plaque microperforé.

2. Composant selon la revendication 1, **caractérisé en ce que** le matériau photocatalytiquement actif se compose de la forme photocatalytiquement active du dioxyde de titane ou contient celle-ci.

3. Composant selon l'une quelconque des revendications précédentes, **caractérisé en ce que** la structure de surface (1) absorbant les sons ou son côté carrière (2) est réalisé(e) de manière structurée pour augmenter la surface photocatalytiquement active et présente à cet effet des nervures ou des moulures.

4. Composant selon l'une quelconque des revendications précédentes, **caractérisé en ce qu'**au moyen de dispositifs de transport d'air (4), par exemple des ventilateurs, une alimentation active en air à traiter peut avoir lieu dans l'espace carrière derrière la structure de surface (1) absorbant les sons.

5. Composant selon l'une quelconque des revendications précédentes, **caractérisé en ce que** la structure de surface absorbant les sons (1) présente des ouvertures supplémentaires afin de garantir une plus faible perte de pression lors de l'écoulement à travers elle.

6. Composant selon l'une quelconque des revendications précédentes, **caractérisé en ce que** les sources de lumière UV (3) sont par exemple des diodes électroluminescentes ou les sources de lumière UV (3) irradient en même temps de la lumière visible pour un éclairage direct ou indirect.

7. Composant selon l'une quelconque des revendications précédentes, **caractérisé en ce que** la structure de surface (1) absorbant les sons peut être chauffée et/ou refroidie ou dispose d'une couche interne pouvant être chauffée ou refroidie (7).

8. Composant selon l'une quelconque des revendications précédentes, **caractérisé en ce que** la structure de surface absorbant les sons (1) est transparente et le matériau photocatalytiquement actif est appliqué sur une surface partielle sur son côté arrière (2).

9. Composant selon l'une quelconque des revendications précédentes, **caractérisé en ce que** la surface limitant l'espace opposé au côté arrière (2) de la structure de surface (1) absorbant les sons est régalement revêtue d'un matériau photocatalytiquement actif ou est par exemple métallisée sous forme de surface réfléchissante.

10. Composant selon l'une quelconque des revendications précédentes, **caractérisé en ce que** le composant fait partie d'un canal (8) ou d'un espace d'une installation de ventilation.

11. Composant selon l'une quelconque des revendications précédentes, **caractérisé en ce que** le composant peut être appliqué ou est appliqué sur des surfaces de limitation d'espace dans des bâtiments.

12. Composant selon l'une quelconque des revendications 1 à 10, **caractérisé en ce que** le compostant peut être rappliqué ou est appliqué dans l'espace interne ou le compartiment moteur de moyens de transport, par exemple sous forme de partie de l'habillage.

13. Composant selon l'une quelconque des revendications 1 à 10, **caractérisé en ce que** le composant fait partie d'un encapsulage ou d'une enceinte de machines ou d'appareils.
